Europäisches Patentamt

(19) European Patent Office

Office européen des brevets

(11) Publication number: **0 172 616**
**A2**

## (12) EUROPEAN PATENT APPLICATION

(21) Application number: **85304157.2**

(51) Int. Cl.⁴: **G 01 N 27/00**

(22) Date of filing: **12.06.85**

(30) Priority: **14.06.84 GB 8415236**

(43) Date of publication of application:
**26.02.86 Bulletin 86/9**

(84) Designated Contracting States:
**AT BE CH DE FR GB IT LI LU NL SE**

(71) Applicant: **THE UNIVERSITY OF SHEFFIELD**
**Western Bank**
**Sheffield, S10(GB)**

(72) Inventor: **Brown, Brian Hilton**
**9 Lorne Close Dronfield Woodhouse**
**Sheffield S18 52J(GB)**

(72) Inventor: **Barber, David Charles**
**26 Westwood Road**
**Sheffield S11 7EY(GB)**

(74) Representative: **Hulse, Thomas Arnold et al,**
**Hulse & Co. Cavendish Buildings West Street**
**Sheffield S1 1ZZ(GB)**

(54) Tomography.

(57) A method for the construction of tomographic images of a body comprises placing an array of spaced electrodes in contact with the body, causing currents to flow in the body, by applying an electrical potential between pairs of electrodes in turn, calculating the potentials between other pairs of electrodes on the assumption that the body consists of one uniform medium, plotting the isopotentials corresponding to the calculated results to create an assumed image of the body, measuring initial potentials between those other pairs of electrodes in sequence over the array of electrodes, measuring subsequent potentials between the same pairs of electrodes in the sequence after a change in the internal state of the body, determining the ratios between the initial potentials and the subsequent potentials in each case, and modifying the image by back projecting the respective ratios along the appropriate isopotentials and thereby increasing the impedance along an isopotential in proportion to a ratio greater than unity or decreasing the impedance in proportion to a ratio less than unity.

EP 0 172 616 A2

Croydon Printing Company Ltd

TOMOGRAPHY

This invention relates to tomography and is concerned with the provision of a method of obtaining tomographic images of a body or a portion thereof (hereinafter referred to simply as a body), more particularly - but not exclusively - of any part of a live human body.

The success of X-ray computed tomography has encouraged the proposal of other medical imaging techniques not fraught with the dangers of X-rays. The use of low frequency electric currents has been suggested although no practical results have been published, but some literature exists on methods which involve measurements of impedance between electrodes on the surface of the body and propose methods for reconstruction of spatial impedance variations. GB-PS 2 119 250A gives examples to which may be added 'Electrical impedance imaging of the thorax' by Y.Kim, J.G. Webster and W.J. Topkins (Jn. of Microwave power, 18(3), 245-257, 1983): 'Fundamental study on electrical impedance CT algorithm utilising sensitivity theorem on impedance

plethysmography', by K. Nakayama, W. Yagi and S. Yagi (Proc. of Vth (Int. Conf. on Electric Bio Impedance), Tokyo, 99-102, 1981): 'A fundamental study of an electrical impedance CT algorithm', by K. Sakamoto and H. Kanai. (Proc. of VIth ICEBI, Zadar, Yugoslavia, 349-352, 1983): and 'Methods and feasibility of estimating impedance distribution in the human torso', by Y. Yamashita and T. Takahashi. (Proc. of Vth ICEBI, Tokyo, 87-90, 1981). A current review is given by D. C. Barber and B. H. Brown (Applied Potential Tomography, Jn. of Physics E., 17,723=733, 1984).

GB-PS 2 119 250A also discloses a contrasting method which involves placing a plurality of surface electrodes at spaced intervals on the body, causing currents to flow in the body, and measuring the potential between pairs of electrodes, calculating the potential in each case on the assumption that the body consists of one uniform medium, obtaining the ratio between the measured potential and the calculated potential in each case, calculating the isopotentials expected within the uniform medium between the pairs of electrodes to create an assumed image of the body, modifying the assumed image by back projecting the respective ratios along the appropriate isopotentials, by increasing the impedance along an isopotential in proportion to a ratio greater than unity or decreasing the impedance in proportion to a ratio less than unity, and plotting the modified impedances to create a tomographic image.

The modifying of the impedance distribution in this manner is known as "back projection", and the execution of the back projection (or the superimposition of the modified impedance along isopotentials) results in a tomographic image of the distribution of impedance over the cross-sectional area of the body in the plane containing the electrodes.

The resolution of the tomographic image can be improved by "iteration" (i.e., by recalculating the potentials in each case using the modified impedance distribution as an approximate guide to the actual distribution of impedance, obtaining the ratio between the recalculated potential and the measured potential in each case, and modifying the modified impedance distribution accordingly) and/or by "weighting" the "back projection" ratios in accordance with changing distribution of isopotentials through the body.

Although a computer is used to calculate the potentials, obtain the ratios, and effect the "iteration" and/or "weighting" of the "back projection" ratios, and plotting of the isopotentials is carried out by a visual display unit (VDU) and/or a print-out unit run off the computer, the calculation and plotting of isopotentials, followed by measurements of potentials, "back projection", "iteration" and "weighting", are time-consuming and do not enable rapid changes in the internal state of the body to be seen.

The object of the invention is, therefore, the provision of a method of obtaining tomographic images of a body by causing currents to flow in the body and measuring potentials between pairs of electrodes in an array of spaced electrodes and affording a picture of the changes in the state of the body.

According to the present invention, a method for the construction of tomographic images of a body comprises placing an array of spaced electrodes in contact with the body, causing currents to flow in the body, by applying an electrical potential between pairs of electrodes in turn, calculating the potentials between other pairs of electrodes on the assumption that the body consists of one uniform medium, plotting the isopotentials corresponding to the calculated results to create an assumed image of the body, measuring initial potentials between those other pairs of electrodes in sequence over the array of electrodes, measuring subsequent potentials between the same pairs of electrodes in the sequence after a change in the internal state of the body, determining the ratios between the initial potentials and the subsequent potentials in each case, and modifying the image by back projecting the respective ratios along the appropriate isopotentials and thereby increasing the impedance along an isopotential in proportion to a ratio greater than unity or decreasing the impedance in proportion to a ratio less than unity.

The image produced by this method does not contain the static structures within the body but gives a picture of the internal state of the body after the change of state, which may be effected by means of an injection into the blood stream and/or by ingesting food and/or drink, or which may be the result of respiration, gastric emptying, internal bleeding, or the cardial cycle, and the plotting of the isopotentials may be effected by a visual display unit (VDU) and/or a print-out unit, forming images by intensity of dots in proportion to impedance.

The invention is particularly advantageous in being able to afford a "dynamic" picture of the continually changing state within a body by repeating the potential measuring sequence and potential difference determinations at regular and/or frequent intervals, and recording and/or viewing the successive modified images corresponding thereto. Changes in impedance distribution can be seen even in the presence of very much larger static impedance variations.

A rate of change of state within the body may be obtained by plotting a graph of the intensity of dots corresponding to the organ under investigation.

One method of carrying out the invention and two series of tomographic images obtained thereby will now be described, by way of example only, with reference to the accompanying drawings, in which:-

Figure 1 is a block circuit diagram of equipment used in conjunction with an electrode array indicated diagrammatically round the abdomen of a human body;

Figure 2 is a diagrammatic plan of the electrode array of Figure 1;

Figure 3 is a sequence of print-outs at intervals of 30 seconds with the arrangement of Figures 1 and 2, a drink of 300 ml of tap water being taken before print-out (b);

Figure 4 shows a graph depicting the gastric emptying rate corresponding to the sequence of print-outs of Figure 3, with a corresponding graph derived from gamma camera pictures of the same stomach during the emptying cycle;

Figure 5 corresponds to the left hand end of Figure 1 but shows the electrode array round the torso of the human body; and

Figure 6 is a sequence of print-outs at intervals of 1 second with the arrangement of Figures 1 and 2 as modified by Figure 5 during the respiration cycle.

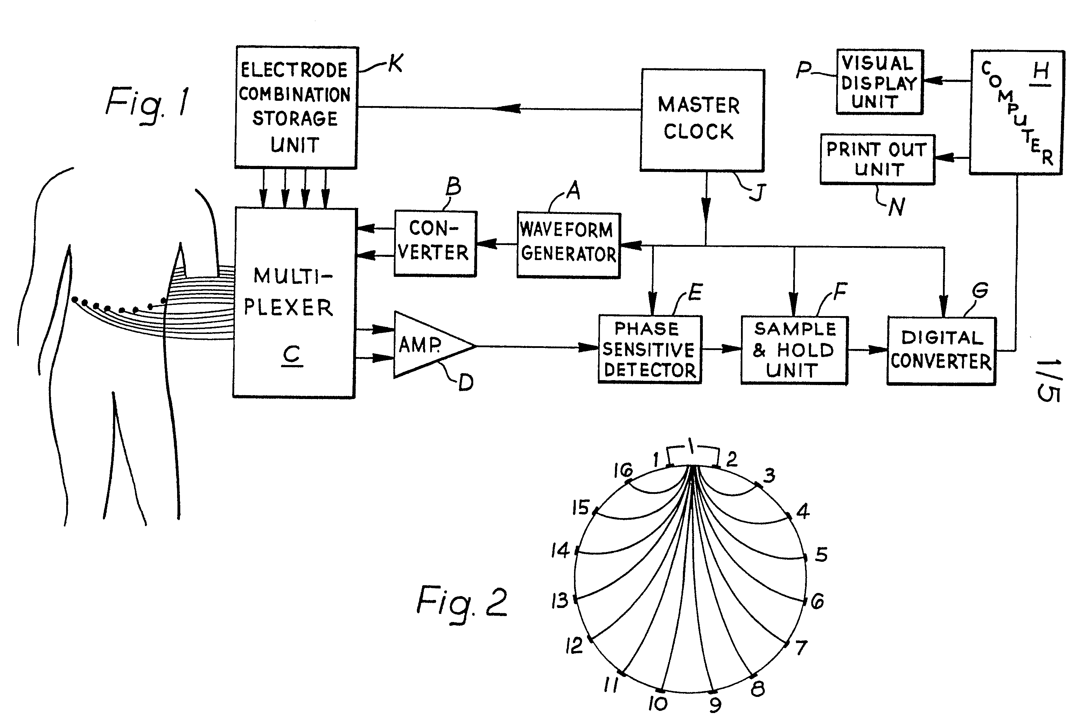

Referring to Figures 1 and 2, an array of sixteen Ag/AgCL electrodes equi-spaced round the abdomen of a human body is coupled to equipment by means of which an applied potential of approximately 3 volts at 4 milliamps is produced by a waveform generator A at 50kHz and applied through a voltage to current, converter B and a multiplexer C in turn between adjacent pairs of electrodes in sequence and in each and every case the resultant potential between every adjacent pair of electrodes is fed through an amplifier D and a phase sensitive detector E and is recorded by a sample and hold unit F, from which the data is fed through a 12-Bit analogue to digital converter G to a computer H. The units A, E, F and G of the equipment are all controlled by a master clock J, which also controls the multiplexer through a unit K which stores the electrode combinations. The sixteen electrodes shown in Figure 2 give rise to 1456 potential measurements which can be recorded in 1.456 seconds or less.

0172616

Figure 2 also shows the assumed image of isopotentials to be expected when current is applied between electrodes 1 and 2, with the body assumed to consist of one uniform medium and circular in cross-section.

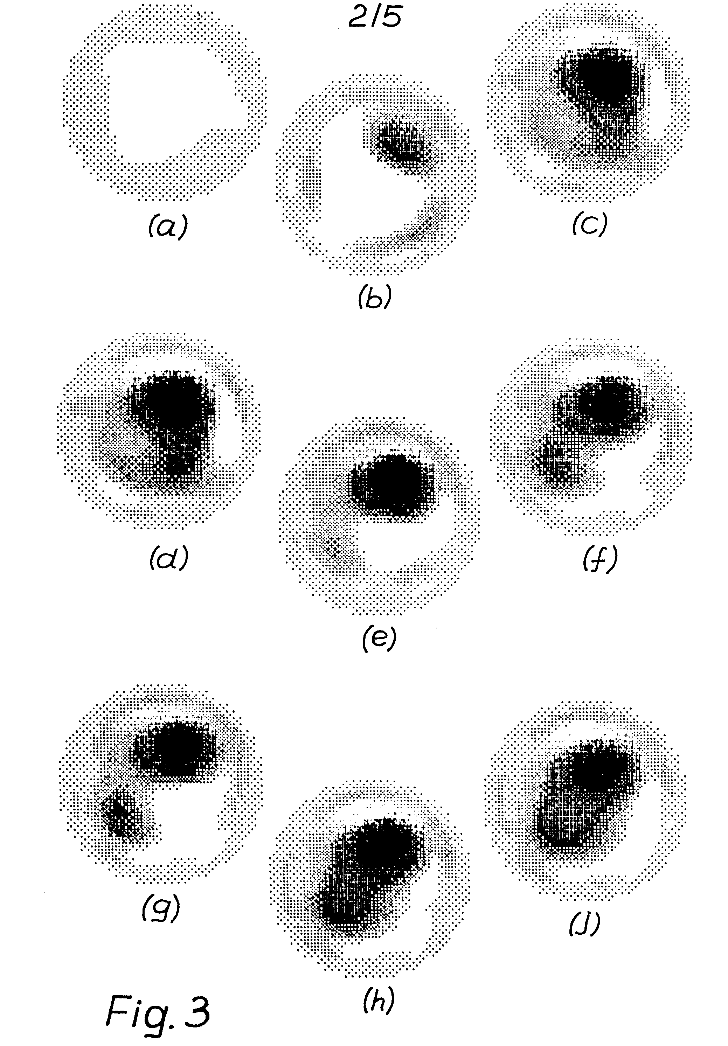

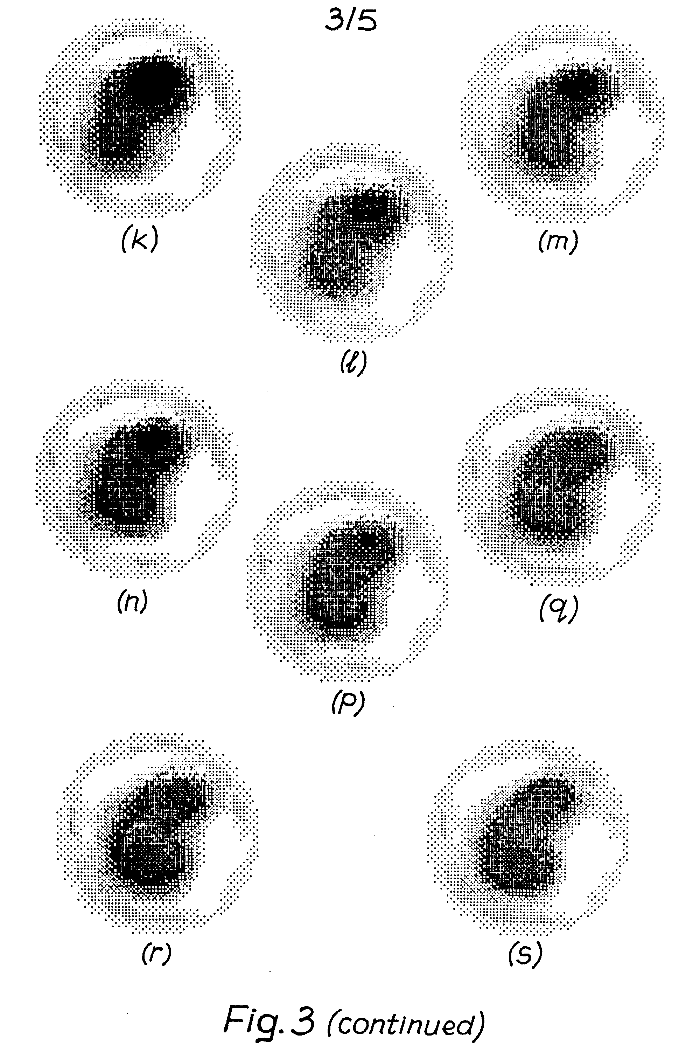

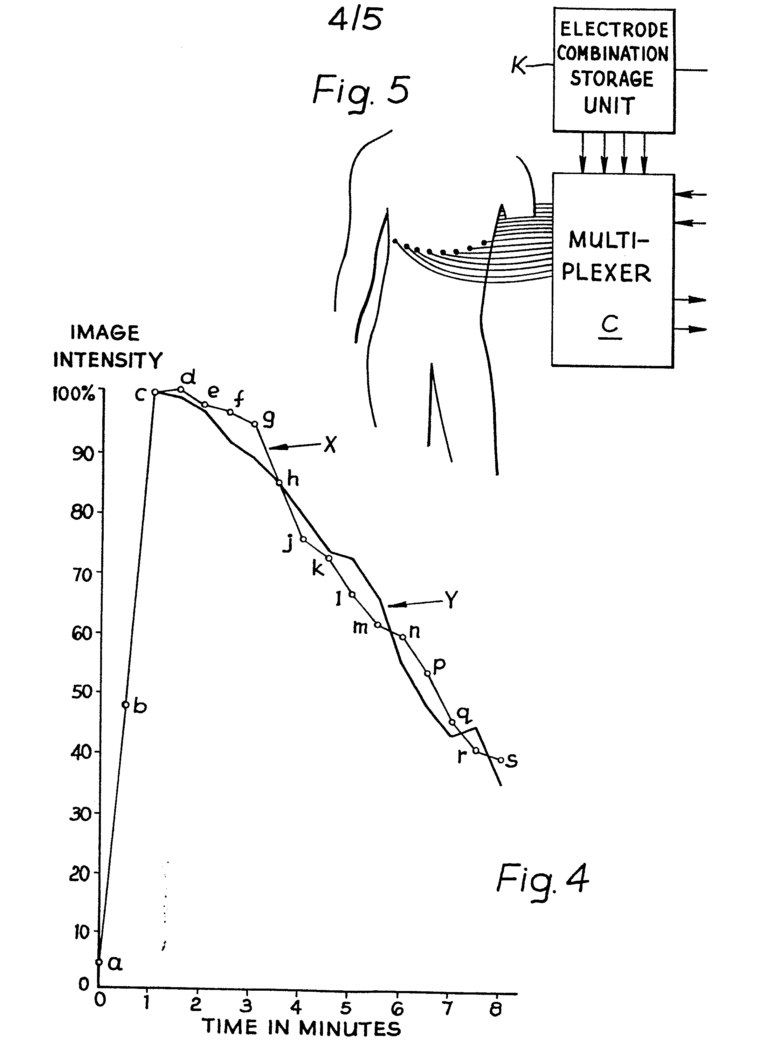

Initial potentials between adjacent pairs of electrodes (other than the pair between which the current is applied) are measured in sequence over the array of electrodes, subsequent potentials between the same pairs of electrodes are measured in the same sequence after a change in the internal state of the body (which in this case results from having a drink - as indicated previously and as will be referred to again presently with reference to Figures 3 and 4), the subsequent potentials are compared by the computer H with the respective initial potentials, and the ratios are back projected along the appropriate isopotentials shown in Figure 2, by increasing the impedance along an isopotential in proportion to a ratio greater than unity or decreasing the impedance in proportion to a ratio less than unity. Thus thirteen back projections can be made for every pair of current drive electrodes (a potential cannot be recorded from a current drive electrode) and the modified impedances along the isopotentials plotted. The plots of the modified impedance along isopotentials are superimposed on those obtained for each and every pair of drive electrodes, by means of the computer linked to a print-out N, to give a back projected tomographic image of the type shown in Figure 3, and to a visual display unit (VDU) P, to give a visually displayed back projected image (not shown).

In Figure 3 the stomach is well outlined following the drink, which is taken before print-out (b). Anterior is on the right and left is at the top of each image. As the changes disappear in the stomach they appear in the small intestine. By taking the maxiumum intensity of image in Figure 3 as 100% it is possible to plot a graph X in Figure 4 showing gastric emptying, which compares with a corresponding graph Y derived from gamma camera pictures of the same stomach during the emptying cycle; but it must be borne in mind that in the latter case the subject or patient has to suffer the discomfort and risk of a radioactive meal in order for the gamma camera pictures to be taken.

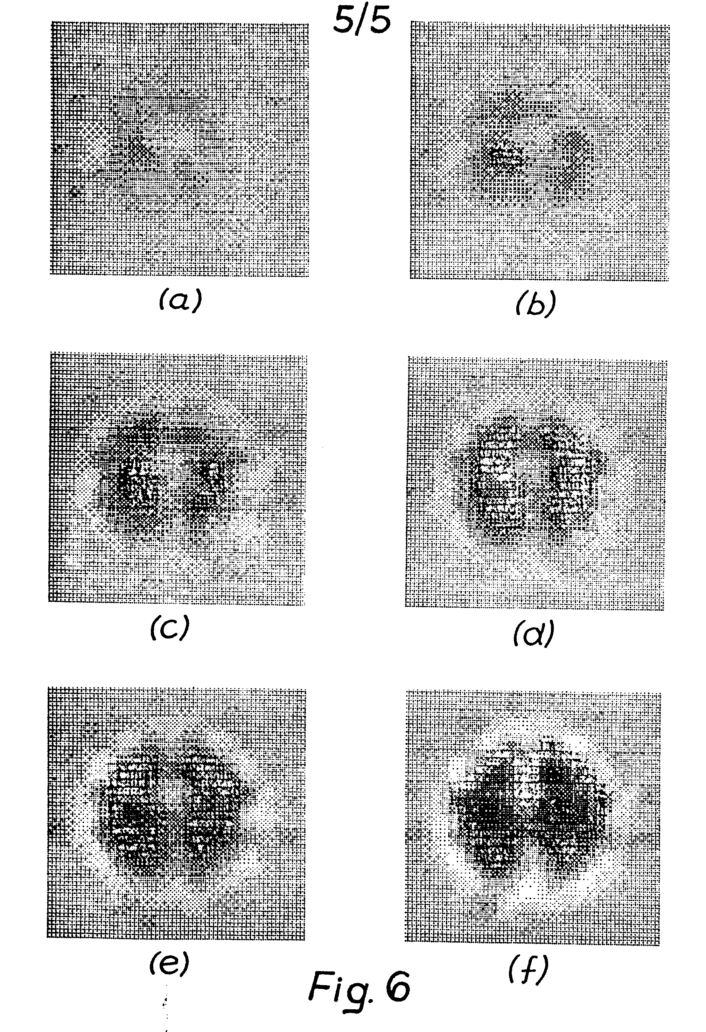

In Figure 5 the sixteen electrodes are disposed round the torso, and Figure 6 shows a resulting sequence of six print-outs at intervals of 1 second during inspiration after inhalation respectively of (a) 0.45, (b) 1.0, (c) 1.5, (d) 2.0, (e) 2.7 and (f) 4.2 litres of air. The lungs are clearly seen, with anterior at the bottom of each image. Any defect in ventilation will show as a distortion of the image of the lungs.

- 12 -

0172616

## CLAIMS

1. A method for the construction of tomographic images of a body comprising placing an array of spaced electrodes in contact with the body, causing currents to flow in the body, by applying an electrical potential between pairs of electrodes in turn, calculating the potentials between other pairs of electrodes on the assumption that the body consists of one uniform medium, plotting the isopotentials corresponding to the calculated results to create an assumed image of the body, measuring potentials between those other pairs of electrodes in sequence over the array of electrodes, and utilising the measured potentials to modify the image, characterised by first measuring initial potentials, then measuring subsequent potentials between the same pairs of electrodes in the same sequence after a change in the internal state of the body, determining the ratios between the initial potentials and the subsequent potentials in each case, and modifying the image by back projecting the respective ratios along the appropriate isopotentials and thereby increasing the impedance along an isopotential in proportion to a ratio greater than unity or decreasing the impedance in proportion to a ratio less than unity.

2.    A method as in Claim 1, characterised in that the potential measuring sequence and potential difference determinations are repeated at intervals, and in that the successively modified images corresponding thereto are plotted, to afford a "dynamic" picture of the continually changing state within the body.

3. A method as in Claim 1 or Claim 2, characterised in that the plotting of the isopotentials is effected by a visual display unit.

4. A method as in Claim 1 or Claim 2, characterised in that the plotting of the isopotentials is effected by a print-out unit forming images by intensity of dots in proportion to impedance.

5.    A method as in Claim 4, characterised in that a rate of change of state within the body is obtained by plotting a graph of the intensity of dots corresponding to the organ under investigation.

Fig. 1

Fig. 2

1/5

0172616

(a)

(b)

(C)

(d)

(e)

(f)

(g)

(h)

(J)

Fig. 3

(k)

(ℓ)

(m)

(n)

(P)

(q)

(r)

(s)

Fig.3 (continued)

0172616

4/5

Fig. 5

ELECTRODE
COMBINATION
STORAGE
UNIT

K

MULTI-
PLEXER

C

IMAGE
INTENSITY

Fig. 4

TIME IN MINUTES

(a)

(b)

(c)

(d)

(e)

(f)

Fig. 6